# EUROPEAN PATENT SPECIFICATION

(11) **EP 1 243 924 B1**
(45) Date of publication and mention of the grant of the patent: **24.11.2004**
(21) Application number: 02006303.8
(22) Date of filing: 20.03.2002
(51) Int. Cl.: G01N 33/543

(54) **Method for the selection of reagents and solid phase components in specific binding assays free of advanced glycosylation endproducts**
Verfahren zum Auffinden von Reagenzien und Festphasenkomponenten in spezifischen Bindungsassays, frei von fortgeschrittenen Glykosylierungsendprodukten
Procédé de criblage de réactifs et composants en phase solide exempts de produits finaux de glycosylation avancée

(30) Priority: 22.03.2001 EP 01107155
(43) Date of publication of application: 25.09.2002
(73) Proprietor: Roche Diagnostics GmbH, 68305 Mannheim (DE); F.HOFFMANN-LA ROCHE AG, 4070 Basel (CH)
(72) Inventor: Kientsch-Engel, Rosemarie, Dr., 82340 Feldafing (DE); Gallusser, Andreas, Dr., 82377 Penzberg (DE); Naser, Werner, Dr., 82377 Penzberg (DE); Lill, Helmut, Dr., 82407 Wielenbach (DE); Stahl, Peter, Dr., 82347 Bernried (DE); Kott, Theresa, 81379 München (DE); Maier, Josef, 82362 Weilheim (DE)

(56) References cited:
- WO-A-96/21450
- US-A- 5 318 982
- US-A- 5 698 197
- PATENT ABSTRACTS OF JAPAN vol. 2000, no. 16, 8 May 2001 (2001-05-08) & JP 2001 004624 A (A & T:KK), 12 January 2001 (2001-01-12)

## Description

The present invention relates to the field of detection and measurement of advanced glycosylation endproducts (AGEs), in particular to methods of selection and/or quality control of reagents or coated solid phase components appropriate for AGE-assays.

Reducing sugars, e.g., glucose or carbonyl groups, have been shown to react non-enzymatically with protein amino groups to form a diverse series of protein bound moieties often with fluorescent and cross-linking properties. These compounds, called advanced glycosylation or advanced glycation endproducts ("AGEs"), have been implicated in the structural and functional alternation of proteins during aging and in certain diseases, e.g., in long-term diabetes. Several AGEs have been identified on the basis of *de novo* synthesis and tissue isolation procedures.

In a first step glucose and other reducing sugars attach non-enzymatically to the amino groups of proteins in a concentration-dependent manner. Over time, these initial Amadori adducts can undergo secondary reactions like further rearrangements, dehydrations and cross-linking with other protein groups to accumulate as a family of complex structures referred to as AGEs.

Substantial progress has been made towards the elucidation of the biological roles and clinical significance of advanced glycosylation endproducts. It is now generally accepted knowledge that many of the conditions heretofore attributed to the aging process or to the pathological effects of diseases such as diabetes, are attributable at least in part to the formation, accumulation and/or activity of AGEs *in vivo*.

Because these secondary reactions occur slowly, proteins may accumulate significant amounts of Amadori products before accumulating a measurable amount of AGEs *in vivo.* These AGEs may modify receptors, all other membrane constituents or enzyme activity. They can cause protein cross-linking, which in turn may reduce the structural and/or functional integrity of organs and organ parts, thus ultimately reducing or impairing organ function.

The advanced glycosylation process is particularly noteworthy in that it particularly effects proteins with long half-lives, such as collagen under conditions of relatively high sugar concentration, such as in diabetes mellitus. Numerous studies have suggested that AGEs play an important role in the structural and functional alteration which occurs in proteins during aging and in chronic disease.

The non-enzymatic reaction between glucose and the free amino groups on proteins leads to a stable amino, 1-deoxy ketosyl adduct, known as the Amadori product. This, e.g., has been shown to occur with hemoglobin, wherein a rearrangement of the amino terminus of the β-chain of hemoglobin by reaction with glucose forms an adduct and gives a product known as hemoglobin A_{1c}. Similar reactions have also been found to occur with a variety of other body protein, such as lens crystalline, collagen and nerve proteins (see Bunn, H. F., et al., Biochem Biophys Res Commun (1975) 103-9; Koenig, R. J., et al., J Biol Chem (1977) 2992-7; Monnier and Cerami, Maillard Reaction in Food and Nutrition, ed. Waller, G. A., American Chemical Society (1983) 431-448; and Monnier and Cerami, Clinics in Endocrinology and Metabolism 11 (1982) 431-452).

Additionally, advanced glycosylation endproducts are noted to form more rapidly in diabetic, galactosemic and other diseased tissue than in normal tissues.

It is also known that oxidation processes, e.g. as encountered under conditions known as "oxidative stress" support processes of AGE-formation (Nawroth, P. P., et al., Med Klin (1999) 29-38).

The "family" of AGEs includes species which can be isolated and characterized by chemical structure, some being quite stable, while others are unstable or reactive. The reaction between reducing sugars and the reactive groups of proteins may initiate the advanced glycosylation process. This process typically begins with a reversible reaction between the reducing sugar and the susceptible group on a protein for instance, to form a Schiff base, which proceeds to rearrange to yield the covalently-bonded Amadori rearrangement product. Once formed, the Amadori product undergoes further non-enzymatic rearrangements and reactions to produce the AGE-modified compound.

Initial attempts to develop anti-AGE antibodies against specific AGE-structures or against AGE-proteins produced *in vitro* lead to antisera not appropriate for detection of AGEs formed *in vivo* (Nakayama et al., Biochem. Biophys. Res. Comm. 162 (1989) 740-745; Horiuchi et al., J. Biol. Chem., 266 (1991) 7329-7332).

Yoshinori, Y., patent abstracts of Japan vol. 2000, no. 16, 8 May 2001 (2001-05-08) & JP 2001 004624 A (A & T:KK), 12 January 2001 (2001-01-12), discloses an immunoassay for AGE based on latex beads sensitized with antibody specifically recognizing AGE.

In WO 96/21450 as well as in US 5,318,982 methods for inhibiting nonenzymatic cross-linking (protein aging) are disclosed.

In WO 93/13421 for the first time antibodies are described which recognize and bind to in-vivo-derived AGEs. These antibodies have been used in competitive immunoassays. The methodological details are not given.

US 5,610,076 describes the specific detection of hemoglobin carrying AGE-structures (Hb-AGE). The improvement described in this patent resides in the pretreatment of samples with detergents and/or chaotropic reagents. The positive effects of such pretreatment are explained by exposure of Hb-AGE epitopes that otherwise would not be accessible to anti-AGE antibodies. BSA-AGE is used as antigen in a competitive immunoassay setting and directly coated to the solid phase of micro titer plates.

US 5,698,197 describes a monoclonal antibody (4G9) reactive with in-vivo formed AGEs. In one embodiment this antibody is used in a sandwich type ELISA to detect Apo-B-AGE, IgG-AGE, collagen-AGE, serum AGE-peptides and proteins as well as urinary-AGE peptides and proteins. In order to perform these AGE-sandwich assays monoclonal antibody 4G9 or an immunoreactive fragment thereof is directly coated to the solid phase. Competitive immunoassay settings are also described using 4G9. BSA-AGE is used and coated to the solid phase of such assays.

A commercially viable assay - amongst other things - requires that results generated using such assays are reproducible from laboratory to laboratory, as well as from lot to lot. Reagent or kit stability during shipment and/or storage are also very important criteria for safe and routine use of AGE-assays.

Despite the significant progress made in generating appropriate (monoclonal) antibodies cross-reactive between *in vitro*-produced and *in vivo*-formed AGEs, and despite the advantageous use of agents exposing AGE-epitopes, no viable commercial AGE-assay has become available.

As already mentioned, AGEs are hall-marks of pathological processes, e.g. as caused by diabetes or conditions of oxidative stress. Therefore, reliable and reproducible routine assays are urgently needed. All AGE-assays known in the art, e.g., from the patent literature mentioned above, make use of direct coating of either a protein-AGE-material produced *in vitro* or of an anti-AGE monoclonal antibody. Despite enormous efforts, it has not been possible so far to establish routine assays based on these approaches.

It therefore was an objective of the present invention to identify and define the reasons for the problems encountered with state of the art methods, to develop approaches which help to overcome these problems and to improve methods for detecting or measuring AGEs. Such improvements of course may as well be used in assays to detect or quantify antibodies to AGEs.

The task to be solved was quite significant since various problems are known from the art of which high lot-to-lot variability, high background reaction and kit stability shall be specifically mentioned. Especially the stability of the solid phase component used in an assay for AGEs is most complex and most critical.

It has surprisingly been found that reagents and/or coated solid phase components used in AGE binding assays as well as reagents used to manufacture same may be "contaminated" with AGEs. With other words, reagents and/or solid phase components contain AGE, or contain structures leading to formation of AGE, to a variable extend. Such AGE-contaminant interferes in an assay for AGE.

The methods developed are used to select reagents or especially to select solid phase components appropriate for AGE binding assays, which are free of AGE and thus can be used in improved AGE binding assays for detection and measurement of AGE.

It has surprisingly been found that it is possible to provide AGE-free reagents and/or solid phase components for AGE binding assays by using the methods described herein. Selection and/or quality control of appropriate reagents and coated solid phase components is now possible.

### Summary of the invention

The present invention provides novel methods and technical approaches to solve the problems so far encountered with AGE-binding assays. Methods are disclosed which allow for selection and/or quality assurance of appropriate reagents or solid phase components, which do not interfere with the AGE-binding of the AGE-binding partner used in an assay for detection or measurements of AGEs.

The invention relates to a method for the selection and/or quality assurance of a reagent or a coated solid phase component used in an assay for detection or measurement of an advanced glycosylation endproduct (AGE) employing at least one AGE-binding partner, characterized in that, said reagents or said solid phase components is tested for AGE content and only such reagent or coated solid phase component is selected, which is not reactive with the AGE binding partner used to perform the assay.

### Detailed description of the invention

The present invention in a first embodiment is directed to a method for the selection and/or quality assurance of a reagent or a coated solid phase component used in an assay for detection or measurement of an advanced glycosylation endproduct (AGE) employing at least one AGE-binding partner, characterized in that, said reagent or said solid phase component is tested for AGE content and only such a reagent or coated solid phase component is selected, which is not reactive with the AGE binding partner used to perform the assay.

A reagent in the sense of the present invention is a biomolecule used in an AGE-assay which may undergo processes leading to advanced glycosylation endproducts (AGEs), e.g., a buffer component like a stabilizing protein, an AGE-binding partner, as well as a member of a specific binding pair used to manufacture the solid phase component. As discussed further above, especially proteins are subject to processes leading to AGEs. It is therefore especially preferred that proteins used as buffer components or for coating or blocking non-specific binding of the solid phase while producing the solid phase component are tested for AGE contaminants. Reagents free of AGE, or at least essentially free of AGE are selected and used.

The term "solid phase materials" is used to describe solid phase or carrier materials routinely used in binding assays. Such materials comprise amongst others micro titer plates, tubes or beads of various polymeric nature like for example glass, latex or plastic materials, membranes used in dry chemistry devices, as well as polymeric carrier materials like carbohydrates, (synthetic) polypeptides.

In the sense of this invention it is preferred to understand and use the term "solid phase component" to comprise any of the fore-mentioned solid phase materials coated with at least one member of a specific binding pair. The skilled artisan will appreciate that there exist many ways to produce such solid phase components, i. e. to coat members of a specific binding pair to carrier materials and optionally to block unwanted non-specific reactions by additionally employing blocking reagents, like, e.g. bovine serum albumin (BSA). It is most preferred to understand solid phase component as the coated solid phase in its final stage, i.e. in the stable and packed form in which it is provided to the customer. The term "selected" is used to emphasize that due to different production processes and storage conditions it is necessary to choose appropriate lots of reagents or solid phase components in order to reliably produce AGE-assays.

Of course, reagents manufactured to comprise AGE, e.g. an AGE antigen material or an AGE standard material is not subject to the selection process as described and claimed.

Preferably, a coated solid phase component for use in an AGE assay, which is free of AGE is selected according to methods of the present invention. It is further preferred to additionally select the solid phase components to be also free of reactive carbonyl groups.

The term "quality assurance" is used to indicate that a product, e.g. a reagent or a solid phase component is assessed, e.g. with respect to its relative amount of AGE contamination.

The method for selection or quality assurance is based on employing at least one AGE binding partner. Typical AGE-binding partners comprise polyclonal antisera and monoclonal antibodies, e.g. as described in WO 93/13421; US 5,610,076 or US 5,698,197.

It is preferred to use specific binding assays based on a polyclonal or monoclonal anti-AGE antibody in the assessment of an AGE in a sample and to use the same or similar assay principals in a method of selection or quality assurance.

As indicated above, binding-assays are well known to the expert in the field. Their use dates back about 30 years (Engvall E. and Perlmann P. (1971), Immunochemistry 8, 871; van Weemen, B.K. and Schuurs A.H.W.M. (1971), FEBS letters 15, 232). Since then enormous progress has been made and methods for carrying out specific binding assays as well as practical applications thereof have become general knowledge to the skilled artisan. Methods and procedures summarized in related text books are herewith included by reference and only few examples shall be specifically mentioned: "Practice and theory of enzyme immunoassays" by P. Tijssen (1990) Elsevier Science Publishers B.V.; and various editions of "Methods in Enzymology", Colowick S.P., Caplan N.O., Eds., Academic Press, dealing with immunological detection methods, e.g. Volumes 70, 73, 74, 84, 92 and 121.

Binding assays may be set up in many different ways. Well-known examples e.g., are homogenous or heterogeneous, competitive and sandwich-type assays. A large variety of detection modes is known and only a few examples shall be specifically mentioned, i.e. enzyme immunoassays (EIAs), radio immunoassays (RIAs), fluorescence polarization immunoassays (FPIAs), (electro-)chemi-luminescence immunoassays ((E)CLIAs) and turbidimetric assays.

Most preferred are binding assays comprising at least one member of an immunological binding pair. In a preferred embodiment the binding pair comprises an AGE and an anti-AGE antibody. Especially preferred are chemically defined AGE-structures and antibodies thereto. Solid phase binding assays for determination of AGE require that one partner of the AGE binding pair is directly or indirectly bound to the solid phase.

In order to directly or indirectly bind one member of an AGE specific binding pair to a solid phase the use of antigen/antibody, or hapten/antibody systems or of the avidin/biotin or streptavidin/biotin system is preferred. Most preferably the streptavidin/biotin system is used. In a preferred embodiment the solid phase component comprises avidin or streptavidin as member of an AGE-independent hapten-like binding pair. It is also preferred to use such a solid phase component in combination with biotinylated material comprising the AGE-structure under investigation.

The term antibody shall be understood as comprising polyclonal, monoclonal and chimaeric antibodies, antibody-like binding partners obtained e.g. by phage display or methods of combinatorial chemistry as well as immunoreactive variants or fragments thereof.

According to standard procedures AGE-binding assays may be construed to detect a variety of AGE-structures, e.g., by using polyclonal antisera raised against proteins "AGEed" *in vitro.* Preferably AGE assays are set up to specifically detect only one or very few preferably chemically characterized AGE-structures. Such assays preferably make use of at least one well characterized component in the AGE-antigen system. Preferably either the AGE-structure is chemically characterized or the antibody used is monospecific or monoclonal.

The problems known from the art have been investigated. It has been found, when analyzing various types of solid phase components, that coated solid phases obtained according to standard procedures or from different commercial sources give rise to rather a high and variable background signal in AGE assays.

Many different standard approaches generally used to improve immunoassays, like variation of buffer conditions, variation of stoichiometries of both biotinylated AGE-antigen as well as anti-AGE antibody have been applied in order to e.g. reduce the background problem. None of these routine procedures worked out for the AGE-assays investigated and high and variable background reaction persisted.

Finally, it was investigated whether the background problem might result from AGE-structures on the solid phase component used in such assays. In order to test for AGE-contamination of solid phase components, competition experiments have been set up essentially using the following three assay components: streptavidin-coated solid phase, synthetic AGE-antigen, and AGE-specific antibody.

These experiments surprisingly confirmed that the solid phase components themselves to a variable extend did contain AGE structures or "AGE-contaminants". This surprising finding was key to the invention. Methods have been developed making it now feasible that appropriate materials or coated solid phase components are selected. As described further below, it has been also possible to establish production processes for solid phase components which are essentially free of AGE. Preferably the solid phase components are not reactive with the AGE binding partner used. Reagents used in the manufacturing of kit components like buffers or solid phase components preferably are also selected to be free of reducing carbonyl groups which otherwise over time would lead to the formation of (interfering) AGE.

Since quite a variety of different AGE structures are known, selection of an appropriate reagent or solid phase component is preferably based on the use of the same AGE-specific binding partner also used in the assay for detection and measurement of the AGE. As mentioned, reagents or solid phase components not reactive with the AGE binding partner used in the assay for AGE are selected.

Low levels of such AGE-contaminants e.g. of coated solid phase components are tolerable. Such material still is termed essentially not reactive, as long as such contamination does not influence the assay results, significantly. The tolerable levels vary dependent on the AGE-binding pair used as well as dependent on the sensitivity of the assay system required. The term "not reactive" is used to describe that a tolerably low, no, or at least essentially no AGE content (or with other words AGE-contamination) has been found using the methods according to the present invention or with methods and procedures equivalent thereto. It is preferred, that no AGE-contamination or essentially no AGE contamination is present. It is most preferred that no AGE is present in the material or in the solid phase component selected.

The methods and procedures as described here and as exemplified in the example section enable the production, selection and quality control of appropriate reagents or coated solid phase components for use in improved, e.g., in commercially viable AGE-assays. Such AGE assays fulfill essential requirements, especially in terms of laboratory to laboratory, lot to lot variability and long term stability. Whereas a variety of methods and techniques can be designed in order to assess the AGE-content of reagents, and/or coated solid phase components, it is preferred to use a method for the selection and/or quality assurance of a reagent or a coated solid phase component used in an assay for detection or measurement of an advanced glycosylation endproduct (AGE) employing at least one AGE-binding partner, which is characterized in that, said reagent or said solid phase component is tested for AGE content and only such a reagent or coated solid phase component is selected, which is essentially not reactive with the AGE binding partner used to perform the assay.

It is well-known that quality and especially sensitivity of most binding assays largely depends on the signal to noise ratio. With other words the higher the specific signal and the lower the system background, the better the assay.

In a preferred embodiment, the method for selection and quality assurance is characterized in that said selection or said quality assurance is made by analyzing and calculating the signal to noise ratio in an AGE binding assay.

Measurement of the AGE-specific signal (=positive signal) is performed by using the AGE-antigen and the AGE-binding partner in the same manner as intended or recommended for the commercial product. System background is determined by performing all essential assay steps in the same manner but omitting the first AGE-binding pair member.

In case of a sandwich-type assay, the positive signal, for example, is measured from an immunological sandwich formed between a first anti-AGE-antibody, an AGE antigen or standard material (i.e. an AGE-carrying molecule) and a second anti-AGE-antibody wherein one of these antibodies is directly or indirectly detectable or labeled. The positive signal is defined as the result obtained using the highest standard of a corresponding commercial product when performing the assay according to the instructions. In case no standard material is provided with the assay, the AGE-concentration used to assess the positive signal is chosen to match the higher end of the measuring range intended or given. The value for the system background is obtained using identical reagents omitting the AGE-antigen. The capturing antibody in a sandwich assay preferably is biotinylated and indirectly bound to a solid phase component comprising avidin or streptavidin matrices.

In case of a competitive assay design the AGE antigen preferably is indirectly bound to the solid phase component. Most preferred the AGE antigen is biotinylated and indirectly bound to a solid phase comprising avidin or streptavidin. In such a competitive assay format the background signal is defined as the signal obtained by using the AGE binding reagent, e.g. a peroxidase labeled anti-AGE-antibody according to the assay instructions but without addition of the indirectly binding AGE antigen and without addition of any competing antigen. This way the system background is determined. The positive signal is obtained using the indirectly binding, e.g., biotinylated antigen and no competing antigen (e.g., O-calibrator is used as sample). The signal to noise ratio in such a competitive assay is calculated by dividing the positive signal of the system by the background signal. An example for such calculation can be found in Example 2.

It is further preferred that the AGE-specific positive signal as obtained in said binding assay is at least 5 times higher as compared to the system background, i.e. that the signal to noise ratio is at least 5.

It is even more preferred that the AGE-specific signal in an AGE-binding assay is at least 10 times higher as compared to the system background.

It is most preferred to use an indirect competitive AGE assay in order to select the appropriate quality of a coated solid phase component.

The signal to noise ratio is one way to select an appropriate reagent and especially to select an appropriate coated solid phase component. It has in addition been found that it is also possible to verify AGE contamination by a different type of competition experiments.

Using anti-AGE antibodies it has been found that some reagents as well as state of the art coated solid phase components react with these antibodies. It has been also found that it is possible to compete out at least part of the background signal encountered with critical reagents or solid phase components by use of a reagent which is known to contain the AGE-structure under investigation. These findings demonstrate that such critical reagents or coated solid phases appear to carry the same or at least similar AGE-structures as the AGE-structure under investigation. This is clear-cut evidence for the existence of AGE-contaminants in quite a few of the critical reagents and/or solid phase components tested.

It has quite surprisingly been found that similar competition type experiments as the ones used to identify AGE-contaminants as major source of problems for AGE assays are very useful in order to select or to assure the quality of a material or a coated solid phase component.

Preferably, the method is characterized in that, in a competitive type AGE assay the background signal of a reagent or a coated solid phase component can not be significantly reduced by the specific AGE-antigen under investigation. In this method the same AGE-antigen as contained in the standard material provided for with the assay and the same antibody is used.

The reagent to be investigated is coated to the same solid phase material as the one used to manufacture the coated solid phase component. This coating is performed by direct adsorption according to routine procedures. In order to select an appropriate reagent lot or an appropriate solid phase component, assay steps are performed as required to conduct a competitive AGE assay. However, no indirect coating with an AGE-antigen is made. Instead, the coated solid phase component or the solid phase material coated with the reagent to be tested is used directly and the competitive potential of the AGE standard material is investigated.

In a preferred embodiment the highest concentration of standard material as supplied in the AGE-assay or the AGE concentration matching the highest point of the measuring range in which this critical material or solid coated phase shall be used, does not significantly reduce the background in such a competitive type AGE assay.

Preferably for an ELISA assay with a positive signal in the range of 1000 mE (milli absorbance units) the reduction in background by the highest AGE calibrator used in the assay is ≤ 100 mE.

Therefore, it is further preferred to select reagents or solid phase components in an ELISA method with a positive signal of ≥ 1000 mE, characterized in that, that less than 100 mE and even more preferred less than 50 mE of the overall background signal can be competed out by the analyzed AGE antigen as contained in the highest standard provided or corresponding to the highest point of the measuring range claimed.

More generally speaking, coated solid phase components are selected for which less than 10% or even more preferred less than 5% of the overall positive signal as obtained in an indirect competitive type AGE assay without any competing antigen, are due to an AGE-contamination of the solid phase. Such contamination preferably is measured as reduction in background signal by aid of the above described competitive type assay.

The present invention provides for a further method of assessing the quality of a reagent. In this method the AGE assay as described (cf. above or example 4) is used. The preparation of a critical reagent is assessed like a sample. Reagents are diluted to 50 µg/ml treated with proteinase K and assayed as described. Preferably reagents containing less than 10 ng/ml AGE (i.e. less than 10ng per 50 µg) are selected. Most preferred are reagents free of AGE, i.e., containing no detectable level of AGE are selected.

Solid phase components in binding assays, especially in immunoassays, are usually produced by coating a member of a specific binding pair to a solid phase. This coating follows standard procedures as described in the art. In general the solid phase to which the member of a specific binding pair is coated is washed between one to five times. Optionally, however, such washing step may be omitted. It is also well-known in the art that in the majority of cases it is very advantageous to use additional reagents to block sticky sites and/or to stabilize the coated binding pair member by a blocking and/or stabilizing reagent. Frequently used are, e.g., BSA, casein, dextran, sugars and or detergents. Usually one reagent solution covering both desired effects is employed.

It is obvious, that with the methods at hand and provided by the present invention it now is possible to select the most appropriate reagents for setting up AGE-binding assays. It is a preferred embodiment according to the present invention, that at least one of the reagents used to manufacture coated solid phase component is an AGE-free reagent which is selected according to the methods of the present invention. Especially preferred the member of a specific binding pair which is coated to the solid phase is free of AGE.

Preferred is a process for the production of a solid phase component which is free of advanced glycosylation endproduct (AGE) for use in an assay to measure said AGE, characterized in that, the reagents used for coating are free of AGE.

The reagents used for coating are very critical for the quality of a coated solid phase component. However, also the reagents contained in the post-coating solution, which is used to prevent unspecific binding and which also may contain compounds stabilizing for example the member of a specific binding pair already coated to the solid phase should be AGE free. It is therefore preferred, that the blocking or stabilizing solution is free of AGE.

It has also been found advantageous to avoid sugar or substances comprising a reducing carbonyl group in reagent solutions, especially in the reagent solutions used to manufacture the solid phase component of an AGE assay. Also the reagent compositions contained in such kit, e.g. incubation buffers, preferably are free of sugars. In a further preferred embodiment solid phase components therefore are produced using sugar-free reagent solutions. Especially the reagents used as means for blocking and/or stabilization of said solid phase component are selected to be free of sugars or other reactive carbonyl species. Sugar-free shall be understood to describe non-interfering sugar-concentration. Preferably, sugar concentrations are below 0,1 %, or reagents are essentially free of reducing sugars.

In general, reducing carbonyl groups, especially the carbonyl functions in sugar molecules or the even more chemically reactive carbonyl groups of molecules like glyoxal or methyl glyoxal, have been found critical in the manufacturing of a reagent composition, e.g., like an incubation buffer, and especially in the manufacturing of a coated solid phase component for an AGE-assay.

Reagents and solid phase components are easily tested for reducing carbonyl functions by routine detection measures. Most conveniently detection of reducing carbonyl is performed by the oxime reaction as described in Pure Appl. Chem. (1979) 1803-1814 or by more sensitive enzymatic procedures. Preferably a reagent or a solid phase component is selected which does not contain measurable levels of reducing sugars. Most preferred a reagent or a coated solid phase component is tested for reducing carbonyl groups using horse liver alcohol dehydrogenase and NADH as substrate. Changes in NADH are measured and correlated to the amount of reducing carbonyl groups present according to routine procedures. In case of micro titer wells the reaction product is transferred to suitable UV-cuvettes and measured.

A process for production of an AGE-free solid phase preferably at least makes use of a stabilizing or blocking solution which is free of reducing carbonyl groups.

Preferably also the reagents and solutions used for coating the member of a specific binding pair to the solid phase are free of reducing carbonyl groups.

Of course, processes combining the testing for AGE and carbonyl groups are also within the scope of the present invention. In such a process for example the critical materials, e.g. the preparation of the binding pair member to be coated are tested for AGE-contamination and the post-coating (=blocking or stabilizing) reagent is tested for carbonyl groups. It is also conceivable to test reagents, as well as solid phase components for both AGE-content and carbonyl content.

A preferred method of quality control for a coated solid phase component comprises both the testing for AGE-contamination and the testing for presence of reducing carbonyl groups.

Described in the present invention is a process for the production of a solid phase component which is free of advanced glycosylation endproduct (AGE) for use in an assay to measure said AGE, comprising the steps of
a) coating the solid phase material with a member of a specific binding pair which is free of AGE,
b) washing the solid phase component and
c) adding a blocking or stabilizing solution free of AGE and free of reducing carbonyl groups.

Another process makes use of a binding pair member free of AGE and free of reducing carbonyl groups and of a post-coating solution free of reducing carbonyl groups.

Further is a process wherein the member of the binding pair is avidin or streptavidin.

Most preferred, the avidin or streptavidin is polymerized as described in EP 331 127.

A coated solid phase component as produced according to a process as described above is described in this invention.

With solid phase components, as produced according to procedures known in the art, major problems have been encountered when using same in AGE-binding assays. As demonstrated in the examples given such problems now can be overcome and solid phase components provided which are free of AGE-contaminants.

Stress stability testing is an independent way of assuring the quality of a coated solid phase component and represents an alternative method according to the invention. The solid phase components as produced according to the processes described have been found to be quite stable under routine storage conditions. E.g. they can be stored at 4°C for 12 month. Also they are stable under stress storage conditions of 37°C for three weeks. Under storage no interfering AGE structures are formed.

A stable coated solid phase component for use in an AGE assay is described in the invention.

Use of an AGE-free solid phase component in specific binding assays for detection or measurement of an AGE antigen is a further described in the invention.

AGE-binding assays may be based on any binding partner capable of binding to AGE-structures. Such binding partners may, e.g. be receptors capable of binding to AGEs, e.g., as described in WO 95/29692. It is however preferred to use immunological binding partners in an AGE-binding assay. In a preferred embodiment AGE-free solid phase components are used in a specific binding assay for detection or measurement of AGE-antigen.

In a further embodiment of this invention, a commercial test kit suitable for use in medical, clinical, or research practice may be prepared. In accordance with the assay techniques discussed above such kits will contain at least an AGE-free solid phase component as well as at least one member of an AGE-binding pair. A preferred embodiment is: A test kit for detection or measurement of AGEs in a sample comprising at least (a) an essentially AGE-free solid phase component (b) a reagent containing an AGE-antigen and (c) a binding partner binding to the AGE of said AGE-containing reagent. Such kit may also contain an AGE-structure in form of standard material or positive control. The kits according to the present invention may additionally contain "peripheral" reagents such as buffers, stabilizers, enzyme substrates, etc.

In a preferred embodiment the solid phase component used in a test kit as described above is a micro titer plate (or micro titer plate composed of strips). Preferably such micro titer plate is coated with streptavidin and one partner of an AGE-binding pair is used in a biotinylated form.

A test kit usable in this invention in a further preferred embodiment comprises a solid phase component selected from the group consisting of micro titer plate, assay tube, test strips and assay beads coated with avidin or streptavidin. Preferably this solid phase material is a micro titer plate or is consisting of micro particles, especially of magnetic micro particles coated with polymerized streptavidin.

The following examples, references, and figures are provided to aid the understanding of the present invention, the true scope of which is set forth in the appended claims.

### Figure 1

### AGE-CML in clinical samples

Values for CML-AGE - measured using an AGE-free solid phase component - in samples taken from healthy volunteers and from patients under dialysis treatment are shown.

### Example 1

### Coating wells of micro titer plates (or strips) for in an indirect, competitive AGE-assay

For all experiments micro titer F8-strips quality Maxisorp (Nunc GmbH, Wiesbaden, Germany) were used as solid phase material
Quality 1: Streptavidin coated strips, standard quality, Roche product no. 1302540
Quality 2: Streptavidin coated strips, high binding quality, Roche product no. 1965905
Quality 3 and 4: Coating was performed with TRSA-Bi (=heat-treated bovine serum albumin in biotinylated form) and homogeneously crosslinked streptavidin (according to EP 0 331 127). After 18 h at room temperature the wells were emptied and refilled with the post-coating solution: Dextran T40 10 g/l (in 10 mM potassium phosphate buffer, pH 7,2) for quality 3 and Dextran T40 10 g/l plus BSA (3 g/l) in the same buffer for quality 4. After 30 min the plates were completely emptied, dried over 3 h at 25 °C, 5 % relative humidity and sealed in airtight bags and stored at 4 to 8°C.
Quality 5:

### 1. Coating

30 mg of polymerized streptavidin (= SA-poly), produced as described in EP 0 331 127, is dissolved in 1 1 40 mM potassium phosphate buffer, pH 7,4. The wells of polystyrol micro titer plates with high binding capacity (NUNC Maxisorp®) are filled will 300 µl of this solution and incubated for 18 h at room temperature and the solution removed afterwards.

Greiner Makrolon 600, or Costar high bound solid phase materials have also been tested and found to work as well.

### 2. Washing

All wells are filled with 300 µl 100 mM potassium phosphate buffer, pH 7,2 containing 0,0025 % (w/v) Thesit (W. Kolb AG, Hedingen, Switzerland:"Sympatens-AL/090 P") and incubated for 1 h at room temperature. The plate is emptied and the washing procedure repeated once again.

### 3. Post-coating

All wells are filled with 300 µl bovine serum albumin (Roche Diagnostics GmbH, product no. 1 726 536), 3 g/l in 50 mM potassium phosphate buffer, pH 7,2 and incubated for 1 h at room temperature. The solution is removed by suction.

### 4. Drying

The plates are dried during 4 h in a climatic chamber at 25 °C with 5% relative humidity. Afterwards the plates are sealed in an airtight cover and stored at 4 - 8 °C.

**Table 1:**

| Overview over different coated solid phase components tested | | | | |
|---|---|---|---|---|
| quality | Roche product no. | coating | post-coating | remark |
| 1 | 1302540 | TRSA-SA | BSA + sucrose | standard quality |
| 2 | 1965905 | TRSA-Bi / SA(poly) | BSA + sucrose | high binding quality |
| 3 | - | TRSA-Bi/ SA(poly) | dextran | |
| 4 | - | TRSA-Bi / SA(poly) | BSA + dextran | |
| 5 | - | SA(poly) | BSA | new quality for age assay |
| TRSA = thermo-BSA SA = streptavidin SA(poly) = poly-streptavidin TRSA-Bi = biotinylated thermo-BSA BSA = bovine serum albumin | | | | |

### Example 2

### AGE-CML Determination

1. Preparation and biotinylation of AGE-modified bovine serum albumine
   Bovine serum albumin (BSA, Calbiochem, Order no. 12657) has been subjected to advanced glycation in vitro. For this purpose, BSA (50 mg/ml in 50 mmol/l potassium phosphate, 150 mmol/l sodium chloride, 20 µmol/l coppersulfate, pH 7,4) was incubated in the presence of D-glucose (0,5 mol/l) for 3 weeks at 35 °C. D-glucose was removed by extensive dialysis against 50 mmol/l potassium phosphate buffer, 100 mmol/l sodium chloride, pH 7.5.
   Biotinylation of BSA-AGE was performed in 100 mmol/l potassium phosphate buffer, 100 mmol/l sodium chloride, pH 8.0 using D-Biotinoyl-[epsilon]-aminocaproic acid-N-hydroxysuccinimide ester at a challenge ratio of 10:1. The reaction was stopped after 90 min by the addition of lysine-monochloride to yield a final concentration of 10 mM. The excess label was removed by dialysis against 50 mmol/l potassium phosphate, 150 mmol/l sodium chloride, pH 7,5. The biotinylated product is called "Bi-BSA-AGE".
2. Reagents and solutions used in the ELISA procedure
   2.1. Incubation buffer: Incubation buffer from the Roche FM assay (Order No. 565 440) is used.
   2.2. Bi-BSA-AGE: This biotinylated AGE-antigen is diluted to 1 µg/ml in incubation buffer
   2.3. Standards: 6-(N-carboxymethylamino) caproic acid (=CML), di-sodium salt (MW 233), is used as standard material. Solutions of 0 / 6,25 / 12,5 / 25 / 50 / 100 ng/ml in incubation buffer are prepared. (The solution with 0 CML is also termed 0-calibrator.)
   2.4. Conjugate: Monoclonal anti-CML, clone 4G9, conjugated to horse-radish peroxidase is diluted to 90 mU/ml in incubation buffer
   2.5. Wash medium: A solution comprising 10 mmol/l TRIS/HCl, 150 mmol/l NaCl, 0,001 % (w/v) N-methylisothiazolon, 0,01 % (w/v) 2-chloracetamide, 0,05 % (v/v) Tween-20, pH 7,4 is used as washing buffer.
   2.6. Substrate: 2,2'-azino-di[3-ethylbenzthiazoline sulfonate (6)]. Roche Biochemicals, Order no. 1 684 302 is used as substrate.
   2.7. Micro titer "plate": SA-coated solid phase components 1-5 produced according to example 1.
3. ELISA-procedure
   3.1. Binding of Bi-BSA-AGE (solution 2.2): 100 µl/well are added and incubated for 1 h with shaking at room temperature.
   3.2. Wash 3 times with wash medium (300 µl of solution 1.5 per well). Especially after the last washing step, the plate is tapped thoroughly (upside down) onto blotting paper to remove all remaining liquid.
   3.3. Simultaneous incubation of sample and conjugate:
      The assay is performed using double determinations. 50 µl of unknown sample, standard or control sample, respectively, are pipetted per well, then immediately afterwards 50 µl of conjugate (solution 2.4) per well is added and the mixture is incubated for 1 h with shaking at room temperature.
   3.4. Wash 3 times, as above
   3.5. Substrate incubation: 100 µl of substrate (solution 2.6) per well are added and incubated for 15 to 30 min with shaking at room temperature.
   3.6. Absorbance is measured with a micro titer photometer at 405 nm. Mean values of double or multiple determinations are calculated and CML contents of samples are read off the calibration curve and results given in Tables 2 and 3.

**Table 2:**

| Absorbance with Bi-BSA-AGE (read after 30' of reaction) | | | | | |
|---|---|---|---|---|---|
| CML [ng/ml] | quality of coated solid phase component | | | | |
| | 1 | 2 | 3 | 4 | 5 |
| 0 | 1.676 | 0.696 | 1.318 | 0.75 | 1.419 |
| 6.25 | 1.403 | 0.528 | 1.101 | 0.617 | 1.249 |
| 12.5 | 1.222 | 0.458 | 1.041 | 0.53 | 1.043 |
| 25 | 1.023 | 0.335 | 0.789 | 0.423 | 0.818 |
| 50 | 0.723 | 0.223 | 0.612 | 0.293 | 0.700 |
| 100 | 0.583 | 0.166 | 0.508 | 0.24 | 0.535 |
| competition | 65% | 76% | 61% | 68% | 62% |

**Table 3:**

| Absorbance without Bi-BSA-AGE (read after 30' of reaction) | | | | | |
|---|---|---|---|---|---|
| micro titer solid phase quality | | | | | |
| CML [ng/ml] | 1 | 2 | 3 | 4 | 5 |
| 0 | 0.670 | 0.299 | 0.871 | 0.237 | 0.024 |
| 6.25 | 0.564 | 0.319 | 0.692 | 0.201 | 0.022 |
| 12.5 | 0.458 | 0.316 | 0.582 | 0.159 | 0.016 |
| 25 | 0.385 | 0.294 | 0.387 | 0.113 | 0.005 |
| 50 | 0.338 | 0.268 | 0.333 | 0.094 | 0.009 |
| 100 | 0.273 | 0.300 | 0.238 | 0.081 | 0.008 |
| signal/noise* | 2.5 | 2.3 | 1.5 | 3.2 | 60.4 |

| | | | | | |
|---|---|---|---|---|---|
| (* = absorbance of standard 0 with Bi-BSA-AGE /absorbance of standard 0 without Bi-BSA-AGE) | | | | | |

As it becomes obvious from Table 2, CML is capable of competing with Bi-BSA-AGE for the binding sites of monoclonal antibody 4G9.

Striking differences however, are seen in comparing background signals and competition in streptavidin coated plates of various quality. In Table 3 absorbance values are given without addition of biotinylated bovine serum albumin-AGE. Varying and high levels of background activity are found in the plates produced according in the state of the art procedures. This background reactivity can be effectively competed away by addition of CML. This implies that the solid phase components offered in qualities 1 - 4 are contaminated with AGE-structures.

The striking advantages of the new material (No. 5 in tables 2 and 3) becomes obvious from the signal to noise ratios which are obtained by comparing values of 0-calibrator without Bi-BSA-AGE and with Bi-BSA-AGE. Whereas the novel solid phase quality in the above experiments has been found to result in a signal to noise ratio of 60 the other examples known from the art only exhibit signal to noise ratios from 1.5 to 3.2.

### Example 3

### Stability of the novel streptavidin coated solid phase component

Micro titer wells of quality 5, which were kept for 3 weeks at 4 - 8°C, room temperature, or at 37°C, respectively, were compared in the AGE-CML assay. The specific signal was measured using Bi-BSA-AGE, whereas the unspecific signal was detected using buffer only instead of biotinylated antigen.

**Table 4:**

| ELISA results obtained after different modes of storage for solid phase component number 5 | | | | |
|---|---|---|---|---|
| standard | absorbance at 405 nm | | | |
| CML [ng/ml] | mictro titer wells, 3 weeks at room temperature | | Mictro titer wells, 3 weeks 37°C | |
| | with Bi-BSA- AGE | without Bi-BSA-AGE | with Bi-BSA-AGE | without Bi-BSA-AGE |
| 0 | 1.789 | 0.036 | 1.697 | 0.102 |
| 6.25 | 1.570 | 0.034 | 1.466 | 0.086 |
| 12.5 | 1.401 | 0.034 | 1.255 | 0.071 |
| 25 | 1.022 | 0.029 | 0.996 | 0.057 |
| 50 | 0.907 | 0.032 | 0.827 | 0.055 |
| 100 | 0.691 | 0.032 | 0.638 | 0.049 |

Overall competition using Bi-BSA-AGE was comparable: 61 % or 62 %, respectively, after the two different storage conditions shown. With plates stored at 4°C similar results were obtained.

As can be seen from table 4, the unspecific signal (without Bi-BSA-AGE) raised slightly under stress storage at 37 °C for three weeks, but did not reach critical background values. The high background values observed with the other coated solid phase components 1 - 4 as known from the art (cf.: Example 1 and 2) are very different even when compared to the stressed coated solid phase component. The signal/noise ratio after stress storage still has been found to be 17 as compared to about 1.5 to 3.2 for material known from the art.

Stability under storage "stress" conditions of 3 weeks at 37 °C is known to be equivalent to storage conditions at 4 °C for at least 12 months. With other words the novel solid phase material, even after extended storage at 37 °C for 3 weeks (equivalent to long term storage at 4 °C) are drastically better as solid phase materials produced according to standard procedures ( cf. Solid phase components 1 - 4 in Example 2). The novel solid phase component is also characterized in that even after extended storage or storage under stress conditions a signal to noise ratio of ≥ 10 is found.

### Example 4: Measurement of CML-AGE in clinical samples

The wells of a streptavidin-coated micro titer plate are incubated with Bi-BSA-AGE (at a concentration of 1 µg/ml in incubation buffer). 100 µl/well are incubated at room temperature for 1 hour. Any unbound Bi-BSA-AGE is removed by washing all wells with 3 x 300 µl washing solution.

Sample and peroxidase-conjugated monoclonal antibody are co-incubated. To each well 50 µl of sample, pre-treated by proteinase K, or standard material, is added, immediately followed by 50 µl of conjugate solution prepared as described in example 2. This mixture is incubated for 1 hour at room temperature. Non-bound reagents are removed by washing, as described above.

Proteinase K works over a broad range of concentrations and incubation times. Optimal results are obtained by adjusting the conditions for proteinase K pre-treatment to match the protein content of the sample used.

In case the sample is CSF, the proteinase K pre-treatment is performed by incubating 60 µl of sample with 5 µl of proteinase K-solution (final concentration 1 mg/ml). Reagents are mixed and incubated for 3 hours at 37 °C. To inhibit the proteinase K activity 65 µl of PMSF are added (final concentration 1 mM) and the reagent mixture is further incubated for 30 to 60 minutes. Thereafter, the pre-treated CSF sample is ready for AGE-CML-determination.

In case serum is used as sample, the proteinase K pre-treatment is performed by incubating 10 µl of sample with 100 µl of proteinase K-solution (final concentration 1 mg/ml). Reagents are mixed and incubated for 3 hours at 37 °C. To inhibit the proteinase K activity 100 µl of PMSF are added (final concentration 1 mM) and the reagent mixture is further incubated for 30 to 60 minutes. Thereafter, the pre-treated serum sample is ready for AGE-CML-determination.

Bound peroxidase is detected by standard substrate reaction. 100 µl of substrate solution are added per well and incubated for roughly 30 minutes at room temperature. Peroxidase activity is measured via the change in substrate at a wavelength of 405 nm.

During all incubation steps the reaction mixture in the wells of the micro titer plate is gently moved using a plate shaker device.

Concentration of AGE-CML in the samples measured is extrapolated from the standard curve according to standard procedures.

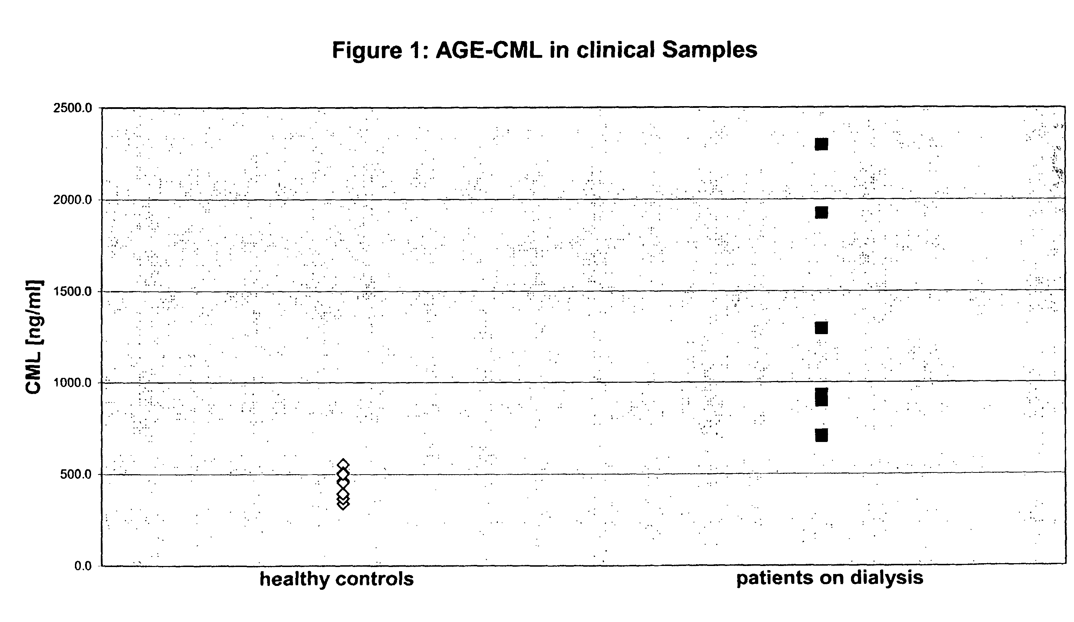

Serum samples from eight healthy controls and from eight patients on dialysis due to diabetic, renal complications have been measured using a microtiter plate produced according to the present invention. Results are summarized in Table 5 and illustrated in Figure 1. It can be seen, that much higher levels of AGE-CML are measured in the diabetic patients.

**Table 5:**

| Results of CML-AGE-measurements in clinical samples | | | |
|---|---|---|---|
| healthy controls | | patients on dialysis | |
| serum sample | AGE-CML [ng/ml] | serum sample | AGE-CML [ng/ml] |
| 1 | 340.3 | 1 | 1922.2 |
| 2 | 368.0 | 2 | 919.4 |
| 3 | 464.6 | 3 | 2295.7 |
| 4 | 552.9 | 4 | 1293.1 |
| 5 | 507.6 | 5 | 707.0 |
| 6 | 455.4 | 6 | 896.9 |
| 7 | 500.6 | 7 | 932.4 |
| 8 | 393.5 | 8 | 701.8 |
| n | 8 | n | 8 |
| mean | 447.9 | mean | 1208.6 |
| standard deviation | 74.3 | standard deviation | 593.2 |

### List of References

Bunn, H. F., et al., Biochem Biophys Res Commun (1975) 103-9
Engvall, E. and Perlman, P., Immunochemistry (1971) 871-4.
Horiuchi, S., et al., J Biol Chem (1991) 7329-32.
Koenig, R. J., et al., J Biol Chem (1977) 2992-7
Monnier, V. M. and Cerami, A., Clinics in Endocrinology and Metabolism (1982) 431-52.
Monnier and Cerami, Maillard Reaction in Food and Nutrition, ed. Waller, G. A., American Chemical Society (1983) 431-448
Nakayama et al., Biochem. Biophys. Res. Comm. 162 (1989) 740-745
Nawroth, P. P., et al., Med Klin (1999) 29-38
Pure Appl Chem (1979) 1803-1814
Tijssen, P. (1990) Elsevier Science Publishers B.V.
van Weemen, B.K. and Schuurs A.H.W.M. (1971), FEBS letters 15, 232
"Methods in Enzymology", Colowick S.P., Caplan N.O., Eds., Academic Press, Volumes 70, 73, 74, 84, 92 and 121.

EP 0 331 127
US 5,610,076
US 5,698,197
WO 93/13421
WO 95/29692

## Claims

1. Method for the selection and/or quality assurance of a reagent, or a coated solid phase component used in an assay for detection or measurement of an advanced glycosylation endproduct (AGE) employing at least one AGE-binding partner, **characterized in that**, said reagent, or said solid phase component is tested for AGE content and only such a reagent, or coated solid phase component is selected, which is not reactive with the AGE binding partner used to perform the assay.

2. The method according to claim 1, further **characterized in that**, said selection or said quality assurance is made by calculating the signal to noise ratio in an AGE binding assay.

3. The method according to claim 1 or claim 2, **characterized in that**, the AGE-specific signal as obtained in said binding assay is at least 5 times higher as compared to the system background.

4. The method according to any of claims 1 to 3, further **characterized in that**, said selection or said quality assurance is performed using a competitive immunoassay for AGE.

5. The method according to claim 1, **characterized in that**, in a competitive type AGE assay the background signal of a reagent, or a coated solid phase component can not be significantly reduced by the specific AGE-antigen under investigation.

## Patentansprüche

1. Verfahren zur Auswahl und/oder Qualitätssicherung eines Reagenz oder einer beschichteten Festphasenkomponente, welche in einem Assay für den Nachweis oder die Messung eines fortgeschrittenen Glykosylierungsendprodukts (AGE) eingesetzt wird, unter Verwendung von wenigstens einem AGE-Bindungspartner, **dadurch gekennzeichnet, dass** das Reagenz oder die Festphasenkomponente auf den AGE-Gehalt getestet wird und nur ein solches Reagenz oder eine solche beschichtete Festphasenkomponente ausgewählt wird, das bzw. die nicht mit dem verwendeten AGE-Bindungspartner reaktiv ist, um das Assay durchzuführen.

2. Verfahren nach Anspruch 1, weiterhin **dadurch gekennzeichnet, dass** die Auswahl oder die Qualitätssicherung durch Berechnung des Signal/Rausch-Verhältnisses im AGE-Bindungsassay erfolgt.

3. Verfahren nach Anspruch 1 oder 2, **dadurch gekennzeichnet, dass** das AGEspezifische Signal, wie im Bindungsassay erhalten, wenigstens 5 mal höher im Vergleich zum Systemhintergrund ist.

4. Verfahren zu einem der Ansprüche 1 bis 3, weiterhin **dadurch gekennzeichnet, dass** die Auswahl oder die Qualitätssicherung unter Verwendung eines kompetitiven Immunassays für AGE durchgeführt wird.

5. Verfahren nach Anspruch 1, **dadurch gekennzeichnet, dass** in der kompetitiven Form des AGE-Assays das Hintergrundsignal eines Reagenz oder einer beschichteten Festphasenkomponente nicht signifikant durch das spezifische AGE-Antigen, das ermittelt wird, verringert werden kann.

## Revendications

1. Procédé pour le choix et/ou l'assurance qualité d'un réactif, ou d'un composant enduit en phase solide utilisé dans un test de détection ou de mesure d'un produit final de glycosylation avancée (AGE) utilisant au moins un partenaire de liaison à l'AGE, **caractérisé en ce que** ledit réactif, ou ledit composant en phase solide est testé pour sa teneur en AGE et seulement un tel réactif, ou composant enduit en phase solide est sélectionné, qui n'est pas réactif avec le partenaire de liaison à l'AGE utilisé pour réaliser le test.

2. Procédé selon la revendication 1, **caractérisé en outre en ce que** ledit choix ou ladite assurance qualité est effectué(e) en calculant le rapport signal/bruit dans un test de liaison à l'AGE.

3. Procédé selon la revendication 1 ou 2, **caractérisé en ce que** le signal spécifique à l'AGE obtenu dans ledit test de liaison est au moins 5 fois supérieur par comparaison à celui de l'arrière-plan du système.

4. Procédé selon l'une quelconque des revendications 1 à 3, **caractérisé en outre en ce que** ledit choix ou ladite assurance qualité est réalisé(e) en utilisant un immunoessaicompétitif pour l'AGE.

5. Procédé selon la revendication 1, **caractérisé en ce que**, dans un test de l'AGE de type compétitif, le signal d'arrière-plan d'un réactif, ou d'un composant enduit en phase solide ne peut pas être sensiblement réduit par l'AGE-antigène spécifique étudié.
